# EUROPÄISCHE PATENTSCHRIFT

(11) **EP 3 380 600 B1**
(45) Veröffentlichungstag und Bekanntmachung des Hinweises auf die Patenterteilung: **26.05.2021**
(21) Anmeldenummer: 17713887.2
(22) Anmeldetag: 14.03.2017
(51) Int. Cl.: C12M 1/00, C12M 3/00, C12M 1/34, F16M 13/02, G01D 11/30

(54) **SONDENHALTER UND VERFAHREN ZUM ANORDNEN EINER SONDE**
PROBE HOLDER AND METHOD FOR POSITIONING A PROBE
PORTE-SONDE ET PROCÉDÉ D'AGENCEMENT D'UNE SONDE

(30) Priorität: 06.06.2016 DE 102016006916
(43) Veröffentlichungstag der Anmeldung: 03.10.2018
(73) Patentinhaber: Sartorius Stedim Biotech GmbH, 37079 Göttingen (DE)
(72) Erfinder: TOPP-MANSKE, Simon, 34253 Lohfelden (DE); ZAHNOW, Christian, 37085 Göttingen (DE); HUSEMANN, Ute, 37079 Göttingen (DE); HUSEMANN, Bernward, 37079 Göttingen (DE)
(74) Vertreter: Vigand, Philippe
(86) Internationale Anmeldenummer: PCT/EP2017/000335
(87) Internationale Veröffentlichungsnummer: WO 2017/211435

(56) Entgegenhaltungen:
- EP-A1- 1 674 835
- EP-A2- 2 829 598
- WO-A1-2011/075036
- US-A1- 2005 005 717

## Beschreibung

Die Erfindung betrifft einen Sondenhalter und ein Verfahren zum Anordnen einer Sonde an einer Anlage für biotechnologische Anwendungen.

Anlagen für biotechnologische Anwendungen wie zum Beispiel Bioreaktoren und Pallettanks dienen zur Aufnahme, zur Lagerung und/oder zum Mischen von biologischen Medien/Kulturbrühen wie z.B. Fluiden und/oder Feststoffen. Dabei können biologische Medien in Behältern wie z.B. Einwegbehältern und/oder Beuteln bereitgestellt werden. Die biologischen Medien werden innerhalb eines solchen Behälters in die Anlage für biotechnologische Anwendungen eingebracht, in der sie gelagert, temperiert und/oder durchmischt werden können. In einer solchen Anlage für biotechnologische Anwendungen können unterschiedliche Untersuchungen an dem biologischen Medium vorgenommen werden.

Dabei erfolgt die Handhabung der Anlage für biotechnologische Anwendungen üblicherweise in einer sterilen Umgebung. Ein Mischen des biologischen Mediums kann dabei mittels eines rotierenden Rührelements erfolgen, das sich innerhalb des Behälters befindet und durch Kraftübertragung von außerhalb des Behälters angetrieben wird.

An der Anlage für biotechnologische Anwendungen kann eine oder mehrere Sonden angeordnet sein, mit der Messungen an dem Medium im Einwegbehälter vorgenommen werden, wie z.B. pH-Wert Messungen etc. Hierbei wird die Sonde an der Anlage für biotechnologische Anwendungen so angeordnet, dass sie z.B. durch ein Fenster mit einem Sondenkopf durch eine Behälterwand des Behälters hindurch in den Behälter eindringt, um so in Berührkontakt mit dem zu untersuchenden Medium zu stehen. Die Sonde kann mit dem Behälter fest verbunden sein.

Hierbei kann das biologische Medium in einem Behälter mit flexiblen Behälterwänden angeordnet sein, insbesondere in sogenannten Einwegbehältern und/oder Einwegbeuteln aus Kunststoff. Beim Befüllen des Behälters mit dem Medium, beim Umrühren des Mediums, und/oder im Laufe des Prozesses kann sich dabei die flexible Behälterwand des Behälters relativ zur Sonde bewegen. So kann sich die Behälterwand beispielsweise unterschiedlich stark ausdehnen. Die Ausdehnungen der Behälterwand können sich dabei einige Zentimeter unterscheiden. Dies führt regelmäßig zu einer Änderungen der Ausrichtung der Sonde, deren Sondenkopf durch eine z.B. vorbestimmte Stelle der Behälterwand hindurch in den Behälter eindringt. Da die Sonde regelmäßig in einem vorbestimmten Winkel und/oder Abstand zum Behälter ausgerichtet sein sollte, kann dabei die durch die Sonde vorzunehmende Messung beeinflusst und/oder verfälscht werden. Weiterhin kann es hierbei zu Beschädigungen am Behälter und/oder der Sonde bzw. der Sondenhalterung kommen.

Das Dokument EP 2 829 598 A2 betrifft einen Sondenverschluss zum Verhindern eines Austretens von Fluid aus einem Bio-pharmazeutischen Kontainer. Hierbei wird ein Sondenverschluss an dem Container befestigt. Eine Sonde wird in den Sondenverschluss eingeführt und dort abgedichtet.

Das Dokument US 2005/0005717 A1 betrifft einen Sondenhalter für einen Feuchtigkeitssensor. Der Feuchtigkeitssensor wird bis an einen Anschlag in den Sondenhalter eingebracht und dort befestigt.

Das Dokument EP 1 674 835 A1 betrifft ein Sondenhalterungssystem zur Wellenüberwachung für Getriebe, Motoren, Einwellen- und Getriebeverdichter.

Der Erfindung liegt die Aufgabe zugrunde, ein Anordnen einer Sonde an einer Anlage für biotechnologische Anwendungen zu verbessern, insbesondere im Hinblick auf die voranstehend bezeichneten Probleme.

Diese Aufgabe wird durch die Gegenstände der unabhängigen Ansprüche gelöst. Bevorzugte Ausführungsformen sind die Gegenstände der abhängigen Ansprüche.

Ein erster Aspekt betrifft einen Sondenhalter zum Anordnen zumindest einer Sonde derart, dass die Sonde zumindest teilweise in eine Anlage für biotechnologische Anwendungen eingreift. Der Sondenhalter weist eine Halterungsbasis zum Anordnen des Sondenhalters an der Anlage für biotechnologische Anwendungen auf und zumindest ein Sondenanordenmittel, das dazu ausgebildet ist, die zumindest eine Sonde so anzuordnen, dass sich die Sonde in eine Sondenerstreckungsrichtung erstreckt. Hierbei ist das Sondenanordenmittel so an der Halterungsbasis angeordnet, dass die Position der Sonde in einer Betriebsposition in Sondenerstreckungsrichtung variabel einstellbar ist.

Der Sondenhalter kann zum Anordnen, Halten, Aufnehmen und/oder Befestigen der Sonde an der Anlage für biotechnologische Anwendungen ausgebildet sein. Insbesondere kann der Sondenhalter zum Aufnehmen einer Mehrzahl von Sonden ausgebildet sein.

Die Erfindung wird nachfolgend mit Bezug auf eine Anlage bzw. Anlage für biotechnologische Anwendungen beschrieben. Diese Anlage kann insbesondere als ein Bioreaktor und/oder als ein Pallettank, also als ein fester Behälter zur Aufnahme eines flexiblen Behälters, ausgebildet sein. Bei der Anlage kann es sich insbesondere um Single-Use-Anlagen für biotechnologische Anwendungen, Crossflow-Anlagen, Bioreaktoren, Biogasanlagen und/oder ähnliche Anlagen handeln.

Die Anlage kann zum Aufnehmen und/oder Lagern eines Einwegbehälters ausgebildet sein, in dem wiederum ein z.B. biologisches Medium angeordnet sein kann und/oder eingefüllt werden kann.

Die Sonde wird so an der Anlage angeordnet und/oder befestigt, dass die Sonde zumindest bereichsweise, insbesondere mit einem Sondenkopf der Sonde, in die Anlage hinein weist. Hierbei kann der Sondenkopf z.B. durch ein Fenster der Anlage hindurch in Berührkontakt mit dem Einwegbehälter stehen. Insbesondere kann der Sondenkopf auch durch eine Behälterwand des Einwegbehälters hindurch weisen und in Berührkontakt mit dem z.B. biologischen Medium stehen, das im Inneren des Einwegbehälters angeordnet ist. Dazu kann an der Wandung des Einwegbehälters eine entsprechende Andockvorrichtung für die Sonde ausgebildet sein. Die Sonde kann somit zumindest einseitig an der Behälterwand befestigt sein und/oder werden.

Die Sondenerstreckungsrichtung kann als eine Sollrichtung ausgebildet sein und eine vorbestimmte Richtung und Position der Sonde in dem Sondenanordenmittel definieren. Die Sonde soll bevorzugt in dieser Sondenerstreckungsrichtung angeordnet sein, wenn sie Messungen an dem Medium durchführt.

Die Sonde kann im Wesentlichen länglich ausgebildet sein und somit eine Längsrichtung aufweisen. Sie kann somit z.B. im Wesentlichen zylinderförmig oder quaderförmig ausgebildet sein. Ist die Sonde korrekt im Sondenanordenmittel angeordnet, kann z.B. die Längsrichtung der Sonde in Sondenerstreckungsrichtung angeordnet sein und/oder dieser entsprechen.

Allgemein kann die Sonde dann in Sondenerstreckungsrichtung angeordnet sein, wenn eine geradlinige Verbindungslinie von einem Sondenmittelpunkt und/oder einem Befestigungsende zu dem Sondenkopf der Sonde, der bei der Messung in Berührkontakt mit dem Medium stehen soll, in etwa mit der Sondenerstreckungsrichtung zusammenfällt. Das Befestigungsende kann hierbei als ein dem Sondenkopf abgewandtes Ende der Sonde ausgebildet sein. Ist die Sonde in Sondenerstreckungsrichtung angeordnet, kann somit eine Wirkrichtung, Messrichtung und/oder Eindringrichtung der Sonde mit der Sondenerstreckungsrichtung als Sollrichtung im Wesentlichen zusammenfallen.

Eine bevorzugte Sondenerstreckungsrichtung ist dabei eine Richtung, die in etwa von 0° bis etwa 30°, insbesondere um etwa 5° bis etwa 20°, geneigt zu einem Lot auf die der Sonde zugewandte Außenfläche der Anlage angeordnet ist. Regelmäßig werden Sonden an einer Anlage für biotechnologische Anwendungen um einen solch kleinen Winkel geneigt zum Lot auf die der Sonde zugewandte Außenfläche angeordnet. Hierbei ist das Lot auf den Bereich der Außenfläche der Anlage gemeint, durch den die Sonde in die Anlage eingreift. Greift die Sonde also z.B. durch eine im Wesentlichen vertikal angeordnete Außenfläche hindurch in die Anlage ein, so kann die Sondenerstreckungsrichtung in etwa von 0° bis etwa 30° geneigt zu einer Horizontalen angeordnet sein.

Die Sonde kann als ein Sensor ausgebildet sein. Dieser Sensor kann z.B. zur Messung eines oder mehrerer der folgenden Eigenschaften des Mediums ausgebildet sein:
- pH-Wert,
- CO2 Gehalt,
- O2 Gehalt,
- Methanol Gehalt,
- Glukose Gehalt,
- Laktat Gehalt,
- Anteil an Biomasse,
- Lichtstreuung,
- elektrische Kapazität, und
- Leitfähigkeit.

Als Sonde kann auch ein Spektroskop im Sondenhalter angeordnet werden.

Der Sondenhalter kann insbesondere dazu ausgebildet und vorgesehen sein, eine Sonde aufzunehmen und/oder an der Anlage anzuordnen, die zumindest einseitig fest mit dem Einwegbehälter verbindbar ist.

Die Sonde kann so in dem Sondenanordenmittel angeordnet werden oder sein, dass sich die Sonde in die z.B. vorbestimmte Sondenerstreckungsrichtung erstreckt. Hierbei kann z.B. die Längsrichtung und/oder die Wirkrichtung etc. der Sonde in Sondenerstreckungsrichtung angeordnet sein. Das Sondenanordenmittel kann sowohl zum Aufnehmen, Anordnen, Halten, und/oder Befestigen der Sonde in Sondenerstreckungsrichtung ausgebildet sein. Insbesondere kann das Sondenanordenmittel z.B. als eine Klammer ausgebildet sein, in die die Sonde eingebracht und aufgenommen werden kann.

Die Halterungsbasis kann als Halterung für das Sondenanordenmittel ausgebildet sein. Hierbei kann die Halterungsbasis unmittelbar an der Anlage befestigt und/oder befestigbar sein. Die Halterungsbasis kann z.B. an und/oder benachbart zu einem Fenster oder einem Rand der Anlage angeordnet werden. Dafür können an der Anlage entsprechende Befestigungsmittel wie z.B. Schienen, Systemschienen, Griffe und/oder Handläufe vorgesehen sein. Die Halterungsbasis kann z.B. über einen Formschluss, eine Schraubverbindung und/oder einem Klemmsitz an der Anlage befestigt werden. Die Halterungsbasis kann zumindest teilweise anlagenfest ausgebildet sein, d.h. zumindest teilweise so an der Anlage befestigbar sein, dass es sich mit diesem Teil relativ zur Anlage im Wesentlichen nicht mehr bewegt.

Das Sondenanordenmittel ist so an der Halterungsbasis angeordnet und/oder befestigt, dass es relativ zur Halterungsbasis (und somit in einer Betriebsposition auch relativ zur Anlage) in Sondenerstreckungsrichtung beweglich ist. Insbesondere ist die Position des Sondenanordenmittels und somit einer daran angeordneten Sonde in Sondenerstreckungsrichtung variabel einstellbar. Dazu ist das Sondenanordenmittel so an der Halterungsbasis angeordnet, dass genau diese Relativposition des Sondenanordenmittels zur Halterungsbasis in Sondenerstreckungsrichtung kontrolliert eingestellt werden kann. Hierzu ist eine entsprechende Befestigung der beiden Bauteile des Sondenhalters (also des Sondenanordenmittels und der Halterungsbasis) vorgesehen, die ein Einstellen der Position in zumindest dieser einen Richtung erlauben.

Die Befestigung des Sondenanordenmittels an der Halterungsbasis kann auch mit mehreren einstellbaren Bewegungsfreiheitsgraden ausgebildet sein. Hierbei kann insbesondere eine Einstellung der Position der Sonde in allen drei Bewegungsrichtung ermöglicht sein, insbesondere in einem Zusammenspiel der Einstellung und/oder Positionierung des Sondenanordenmittels und der Halterungsbasis.

Die Einstellung der Position der Sonde und des Sondenanordenmittels in Sondenerstreckungsrichtung ermöglicht es, auf ein Ausdehnen der Wandung des Einwegbehälters reagieren zu können. Die Einstellmöglichkeit in Sondenerstreckungsrichtung ist hierbei zumindest über eine Ausdehnungslänge gegeben, die einer Differenz zwischen einer üblichen Minimalausdehnung des Einwegbehälters und einer üblichen Maximalausdehnung des Einwegbehälters an der Sondenposition entspricht. Die Einstellmöglichkeit kann auch über eine größere Strecke gegeben sein, z.B. über zumindest die zweifache oder dreifache Ausdehnungslänge.

Da sich die Behälterwand des Einwegbehälters üblicherweise im Wesentlichen senkrecht zur Außenfläche der Anlage ausdehnt bzw. zusammenzieht, und die Sondenerstreckungsrichtung üblicherweise lediglich um einen kleinen Winkel (siehe oben) zu der Senkrechten geneigt ist, ist die Sondenerstreckungsrichtung besonders gut dazu geeignet, die Position der Sonde an eine solche Ausdehnung anzupassen.

Durch diese Einstellbarkeit kann auf eine individuelle Ausdehnung reagiert werden, und die Position jeder einzelnen Sonde relativ zur Anlage verbessert und/oder optimiert werden. Hierdurch kann sowohl die Genauigkeit der jeweiligen Messung verbessert werden, als auch ein Beschädigen des Einwegbehälters oder der Sonde reduziert werden.

Gemäß einer Ausführungsform ist das Sondenanordenmittel so an der Halterungsbasis angeordnet, dass sich die Position der Sonde in Sondenerstreckungsrichtung bei Krafteinwirkung in Sondenerstreckungsrichtung automatisch einstellt. Diese Krafteinwirkung in Sondenerstreckungsrichtung kann insbesondere von der sich ausdehnenden und/oder zusammenziehenden Behälterwand des Behälters bewirkt werden. Auch wenn diese Ausdehnung und/oder dieses Zusammenziehen nicht genau in und/oder gegen die Sondenerstreckungsrichtung erfolgt, so bewirkt die Ausdehnungsbewegung und/oder die Zusammenziehbewegung eine Krafteinwirkung mit zumindest einer Richtungskomponente in oder gegen die Sondenerstreckungsrichtung. Ein Ausdehnen und/oder Zusammenziehen des Behälters kann ausreichend sein, diese Krafteinwirkung auf die am Behälter befestigte Sonde zu bewirken. Dies kann dadurch erreicht werden, dass das Sondenanordenmittel im Wesentlichen "lose" und/oder zumindest in Sondenerstreckungsrichtung beweglich (insbesondere zumindest streckenweise frei beweglich) an der Halterungsbasis angeordnet und/oder befestigt ist. Das Sondenanordenmittel kann somit so an der Halterungsbasis angeordnet sein, dass die Halterungsbasis lediglich das Gewicht des Sondenanordenmittels trägt, ohne die Position und/oder die Ausrichtung des Sondenanordenmittels (und der ggf. darin angeordneten Sonde) statisch zu fixieren, zumindest in Sondenerstreckungsrichtung. Eine zumindest streckenweise freie Beweglichkeit des Sondenanordenmittels in zur Sondenerstreckungsrichtung orthogonale Raumrichtungen kann behindert und/oder (z.B. etwa vollständig) verhindert sein. Zusätzlich zur zumindest streckenweise freien Beweglichkeit in Sondenerstreckungsrichtung kann eine zumindest bereichsweise freie Verschwenkbarkeit des Sondenanordenmittels ermöglicht sein.

Die variable Einstellung der Sonde und des Sondenanordenmittels in Sondenerstreckungsrichtung kann bewirken, dass der Abstand der Sonde bezüglich des Behälters (beispielsweise ein flexibler Einweg- und/oder Einmalbehälter), mit welchem die Sonde in Eingriff steht, ungeachtet der Bewegungen der Behälterwand (z.B. Ausdehnung, Zusammenziehen) im Wesentlichen konstant bleibt und sich nicht ändert. Wenn sich die Behälterwand im Verlauf eines Prozesses nach außen und/oder innen wölbt, beispielsweise aufgrund einer zu- und/oder abnehmenden Füllung, kann das Sondenanordenmittel an der Halterungsbasis eine synchrone Bewegung der am Sondenanordenmittel gehaltenen Sonde nach außen bewirken, und zwar insbesondere im gleichen Ausmaß wie sich der Behälter wölbt. Der Abstand der Sonde zur Behälterwand bleibt somit etwa gleich und/oder konstant. Gleiches kann im Wesentlichen für einen bevorzugten Neigungswinkel der Sonde gelten. Dadurch wird eine mechanische Beanspruchung der Behälterwand reduziert, wie im Falle einer herkömmlichen, starr fixierten Sonde, die die Bewegung der flexiblen Behälterwand nicht nachvollzieht. Der Gleichlauf der Bewegung des Sondenanordenmittels samt Sonde mit der Bewegung der Behälterwand kann zudem bewirken, dass eine Eintauchtiefe des Sondenkopfes und/oder einer Sondenspitze in den Innenraum des Behälters im Wesentlichen konstant bleibt.

Gemäß einer Ausführungsform ist das Sondenanordenmittel dazu ausgebildet, das Gewicht der Sonde im Wesentlichen vollständig zu tragen, wenn die Sonde in die Sondenerstreckungsrichtung angeordnet ist, also z.B. wenn die Sonde im Sondenanordenmittel aufgenommen ist. Hierbei trägt das Sondenanordenmittel die Sonde im Wesentlichen vollständig, ohne jedoch die Position der Sonde in Sondenerstreckungsrichtung festzulegen und/oder zu statisch fixieren. Das Sondenanordenmittel ist somit für die Sonde volltragend ausgebildet und kann die beweglich gelagerte Sonde vollständig aufnehmen. Hierzu kann das Sondenanordenmittel die Sonde an zumindest zwei in Sondenerstreckungsrichtung beabstandeten Haltepunkten und/oder Haltebereichen mechanisch kontaktieren, halten und/oder unterstützen. Hierbei kann das Sondenanordenmittel so ausgebildet sein, dass es das Gewicht der Sonde selbst in einer Betriebsposition, in der der Sondenkopf in den Einwegbehälter eingreift, noch zu zumindest etwa 80% des Sondengewichts trägt, bevorzugt zu zumindest etwa 90% des Sondengewichts, besonders bevorzugt zu zumindest etwa 99% des Sondengewichts. Hierdurch kann sowohl ein Aufbauen und/oder in Betrieb nehmen der Anlage vereinfacht werden, als auch eine Belastung des Übergangs von Einwegbehälter auf die Sonde reduziert werden.

Gemäß einer Ausführungsform ist das Sondenanordenmittel so ausgebildet, dass es die Sonde so aufnimmt, dass zumindest ein Sondenkopf der Sonde frei aus dem Sondenanordenmittel heraus weist und in Sondenerstreckungsrichtung in Richtung zur Anlage hin weist. Das Sondenanordenmittel hält die Sonde lediglich an Haltepunkten und/oder Haltebereichen, die vom Sondenkopf beabstandet sind. Hierbei bleibt der Sondenkopf solange "frei schwebend", solange er noch nicht in den Einwegbeutel eingeführt ist. Das Sondenanordenmittel ist dazu vorgesehen, zumindest teilweise in Sondenerstreckungsrichtung von der Anlage beabstandet angeordnet zu werden, so dass es die Sonde in dieser beabstandeten Position mit dem Sondenkopf voran hin zur Anlage und in den Einwegbeutel hinein weisend anordnen und/oder aufnehmen kann.

Gemäß einer Ausführungsform ist das Sondenanordenmittel dazu ausgebildet, die Sonde in einem Formschluss und/oder Klemmsitz aufzunehmen. Hierbei kann das Sondenanordenmittel z.B. als eine Klammer ausgebildet sein, in die die Sonde eingeklemmt werden kann. Insbesondere kann die Sonde gegen einen Rastwiderstand mit dem Sondenanordenmittel verrastet werden. Dadurch wird eine einfach bedienbare Befestigung der Sonde an der Anlage bereitgestellt.

Gemäß einer Ausführungsform weist die Halterungsbasis eine Befestigungsstange auf, an der das Sondenanordenmittel so angeordnet ist, dass die Position der Sonde in Sondenerstreckungsrichtung variabel einstellbar ist. Hierbei kann die Befestigungsstange einen zumindest bereichsweise runden oder eckigem Querschnitt aufweisen. Das Sondenanordenmittel ist an der Befestigungsstange befestigt, z.B. damit verklemmt, verschraubt und/oder über einen Formschluss verbunden. Hierbei kann z.B. über eine Verstellung der Halterungsbasis die Position der Befestigungsstange einstellbar und/oder verstellbar sein. Hierdurch wird eine Einstellung der Position des Sondenanordenmittels und somit der Sonde in eine zusätzliche Richtung ermöglicht, die von der Sondenerstreckungsrichtung verschieden sein kann.

In einer Weiterbildung dieser Ausführungsform ist das Sondenanordenmittel in

Sondenerstreckungsrichtung verschiebbar, um die Befestigungsstange verschwenkbar und/oder entlang der Befestigungsstange verschiebbar an der Befestigungsstange angeordnet. Die Verschiebbarkeit kann z.B. durch einen Formschluss, einen Klemmsitz und/oder eine Führungsschiene ermöglicht werden. Zusätzlich zur Verschiebbarkeit in Sondenerstreckungsrichtung kann das Sondenanordenmittel um die Befestigungsstange verschwenkbar sein, was eine weitere Einstellmöglichkeit der Relativposition der Sonde zur Anlage ermöglicht. Die Befestigungsstange ermöglicht somit auf eine besonders einfach zu realisierende Art und Weise ein Verstellbarkeit und/oder Einstellbarkeit der Position der Sonde in zumindest zwei Freiheitsgrade, nämlich durch Verschiebung in Sondenerstreckungsrichtung und durch Verschwenken um die Befestigungsstange. Alternativ oder zusätzlich kann das Sondenanordenmittel auch entlang einer Stangenerstreckungsrichtung der Befestigungsstange verschiebbar sein, wodurch eine weitere Einstellmöglichkeit bereitgestellt wird.

In einer Weiterbildung der Ausführungsform mit der Befestigungsstange ist die Befestigungsstange zumindest in einem Befestigungsbereich, an dem das Sondenanordenmittel angeordnet ist, im Wesentlichen orthogonal zur Sondenerstreckungsrichtung angeordnet. Der Befestigungsbereich ist hierbei der Bereich der Befestigungsstange, an dem das Sondenanordenmittel angeordnet ist. Die Befestigungsstange kann zumindest bereichsweise im Wesentlichen parallel zu der Außenfläche der Anlage ausgebildet und angeordnet sein, zu dem die Befestigungsstange benachbart angeordnet ist. Dabei kann die Befestigungsstange insbesondere gebogen ausgebildet sein, wobei die Biegung der Befestigungsstange einer Biegung einer gewölbten, z.B. konvexen Außenfläche der Anlage entspricht und/oder folgt. Ist die Befestigungsstange an und/oder benachbart zu einer im Wesentlichen vertikalen Außenfläche der Anlage angeordnet, kann die Befestigungsstange in einer im Wesentlichen horizontalen Richtung angeordnet sein.

In einer Weiterbildung der Ausführungsform mit der Befestigungsstange weist das Sondenanordenmittel ein Befestigungsmittel auf, mit dem es mittels Formschluss und/oder Klemmsitz an der Befestigungsstange befestigt ist. Ein solches Befestigungsmittel, also z.B. eine Befestigungsklammer, ist besonders einfach und/oder komfortabel von einer Bedienperson verstellbar. Hierbei ist die Position des Sondenanordenmittels und somit der Sonde relativ zur Halterungsbasis und somit zur Anlage verstellbar und/oder einstellbar.

In einer Weiterbildung der Ausführungsform mit der Befestigungsstange sind an der Befestigungsstange eine Mehrzahl von Sondenanordenmittel zum Anordnen jeweils zumindest einer Sonde so angeordnet, dass die Position der jeweiligen Sonde in ihrer jeweiligen Sondenerstreckungsrichtung variabel einstellbar ist. Hierdurch wird die Anzahl der Sondenanordenmittel und somit der Sonden erhöht, die an der Anlage angeordnet werden können. Hierbei können die Sondenanordenmittel im Wesentlichen baugleich ausgebildet sein, also insbesondere baugleich zu dem voranstehend beschriebenen Sondenanordenmittel. Insbesondere kann die Befestigungsstange zum Befestigen von zwei, vier oder mehr der Sondenanordenmittel ausgebildet sein, wobei nicht an allen dieser für Sondenanordenmittel vorgesehenen Plätze auch tatsächlich ein Sondenanordenmittel und/oder eine Sonde angeordnet sein muss. Hierbei kann für jedes Sondenanordenmittel eine eigene Sondenerstreckungsrichtung vorgesehen sein. Diese mehreren Sondenerstreckungsrichtungen können sich leicht voneinander unterschieden, also z.B. um einige Grad voneinander abweichen. Hierbei können die Sondenerstreckungsrichtungen der der jeweiligen Sonde zugewandten Außenfläche der Anlage folgen, so dass die jeweiligen Sondenerstreckungsrichtungen jeweils gleich weit zum Lot auf ihre jeweils zugeordnete Auftreffoberfläche der Anlage geneigt angeordnet sein können. Die Nutzung einer Befestigungsstange zur Befestigung mehrerer Sondenanordenmittel und somit mehrerer Sonden ist hierbei besonders effizient und Bauteil-unintensiv.

In einer Weiterbildung der Ausführungsform mit der Befestigungsstange weist die Halterungsbasis zumindest zwei Befestigungsstangen auf, an denen jeweils zumindest ein Sondenanordenmittel zum Anordnen jeweils zumindest einer Sonde so angeordnet ist, dass eine Position der jeweiligen Sonde in ihrer jeweiligen Sondenerstreckungsrichtung variabel einstellbar ist. Die zumindest zwei Befestigungsstangen können z.B. im Wesentlichen parallel zu einer Außenflächen der Anlage zueinander versetzt angeordnet sein, z.B. vertikal übereinander. Die zumindest zwei Befestigungsstangen können im Wesentlichen parallel zueinander angeordnet sein. Hierdurch wird die Anzahl der Sondenanordenmittel und somit der Sonden erhöht, die an der Anlage angeordnet werden können. Für die mehreren Sondenanordenmittel gilt das Gleiche wie in der voranstehenden Weiterbildung beschrieben. So können die Sondenanordenmittel im Wesentlichen baugleich ausgebildet sein, also insbesondere baugleich zu dem eingangs beschriebenen Sondenanordenmittel. Insbesondere muss nicht an allen der für Sondenanordenmittel vorgesehenen Plätze auch tatsächlich ein Sondenanordenmittel und/oder eine Sonde angeordnet sein. Es kann für jedes Sondenanordenmittel eine eigene Sondenerstreckungsrichtung vorgesehen sein. Diese mehreren Sondenerstreckungsrichtungen können sich leicht voneinander unterschieden, also z.B. um einige Grad voneinander abweichen. Hierbei können die Sondenerstreckungsrichtungen der Außenfläche der Anlage folgen, so dass die jeweiligen Sondenerstreckungsrichtungen jeweils gleich weit zum Lot auf ihre jeweils zugeordnete Auftreffoberfläche der Anlage geneigt angeordnet sein können. Wie im Zusammenhang mit der voranstehenden Weiterbildung beschrieben, können auf den unterschiedlichen Befestigungsstangen jeweils mehrere Sondenanordenmittel angeordnet werden.

Gemäß einer Ausführungsform ist die Halterungsbasis so an der Anlage für biotechnologische Anwendungen angeordnet, dass eine Position des Sondenanordenmittels in einer Außenflächenrichtung der Anlage für biotechnologische Anwendungen variabel einstellbar ist, wobei die Außenflächenrichtung etwa parallel zu einer Außenfläche der Anlage für biotechnologische Anwendungen angeordnet ist und einen Winkel von zumindest etwa 45° zu der Sondenerstreckungsrichtung bildet. Hierbei kann die Außenflächenrichtung als eine z.B. ungekrümmte Erstreckungsrichtung der der Sonde zugewandten Oberfläche der Anlage ausgebildet sein, z.B. als eine im Wesentlichen vertikale Richtung. Die Außenflächenrichtung kann in einem Winkel von etwa 45° bis etwa 90° zur Sondenerstreckungsrichtung angeordnet sein, bevorzugt von etwa 65° bis etwa 85° zur Sondenerstreckungsrichtung. So kann die Halterungsbasis z.B. zumindest zweiteilig ausgebildet sein, wobei ein erstes Bauteil der Halterungsbasis an der Anlage befestigt ist, während das Sondenanordenmittel am zweiten Bauteil der Halterungsbasis angeordnet ist. Die beiden Bauteile der Halterungsbasis können gegeneinander in Außenflächenrichtung verschiebbar ausgebildet sein. Dadurch ermöglicht diese Ausbildung der Halterungsbasis eine Einstellung der Position der Sonde relativ zur Anlage um einen weiteren Freiheitsgrad.

In einer Weiterbildung dieser Ausführungsform weist die Halterungsbasis eine Nut und eine in die Nut eingreifende Feder auf, wobei sich die Nut und die Feder in etwa parallel zueinander und parallel zu der der Sonde zugewandten Außenflächenrichtung erstrecken. Eine Verbindung zweier Bauteile der Halterungsbasis über eine einstellbare Nut und Feder-Verbindung stellt eine einfach zu realisierende Möglichkeit bereit, die voranstehend beschriebene Einstellmöglichkeit zu implementieren. Zusätzlich kann die Feder in der Nut festgeschraubt sein.

Ein weiterer Aspekt betrifft eine Anlage für biotechnologische Anwendungen zum Aufnehmen eines Einwegbehälters mit einem Sondenhalter gemäß dem ersten Aspekt, wobei im Sondenhalter die zumindest eine Sonde in Sondenerstreckungsrichtung angeordnet ist. Insbesondere können an der Anlage für biotechnologische Anwendungen mehrere solcher Sondenhalter angeordnet sein, und/oder mehrere Sonden in dem oder den Sondenhalter(n).

In einem Ausführungsbeispiel der Anlage für biotechnologische Anwendungen ist in der Anlage für biotechnologische Anwendungen der Einwegbehälter angeordnet. Ein Sondenkopf der Sonde greift durch eine Behälterwand des Einwegbehälters hindurch ins Innere des Einwegbehälters ein.

Hierbei kann die Sonde z.B. mit ihrem Sondenkopf fest an der Behälterwand des Einwegbehälters befestigt sein. Der Sondenhalter kann dabei das Gewicht der Sonde tragen und dadurch die Ausrichtung der Sonde in zumindest eine etwa vertikale Raumrichtung festlegen und/oder definieren. Die genaue Ausrichtung der Sonde in Sondenerstreckungsrichtung kann hierbei von dem sich ausdehnenden und/oder sich zusammenziehenden Einwegbehälter bestimmt werden, an dessen Behälterwand die Sonde befestigt ist.

Ein weiterer Aspekt betrifft ein Verfahren zum Anordnen zumindest einer Sonde derart, dass die Sonde zumindest teilweise in eine Anlage für biotechnologische Anwendungen eingreift, mit den Schritten:
- Bereitstellen einer Halterungsbasis zum Anordnen eines Sondenhalters an der Anlage für biotechnologische Anwendungen;
- Anordnen einer Sonde in einem Sondenanordenmittel, welches an der Halterungsbasis angeordnet ist, so dass sich die Sonde in eine Sondenerstreckungsrichtung erstreckt; und
- variables Einstellen einer Position der Sonde in Sondenerstreckungsrichtung.

Das Verfahren kann insbesondere mittels eines Sondenhalters gemäß dem ersten Aspekt und/oder an einer der Anlage für biotechnologische Anwendungen gemäß dem zweiten Aspekt durchgeführt werden. Deswegen betreffen all im Zusammenhang mit diesen beiden Aspekten getroffenen Aussagen auch das Verfahren gemäß dem dritten Aspekt und umgekehrt. Das variable Einstellen der Position der Sonde in Sondenerstreckungsrichtung kann hierbei von einer Behälterwand verursacht werden, an der die Sonde befestigt ist.

Die Erfindung wird nachfolgend anhand von in Figuren gezeigten Ausführungsbeispielen näher beschrieben. Einzelne, in den Figuren gezeigte Merkmale können mit anderen Ausführungsbeispielen zu weiteren Ausführungsformen kombiniert werden. Gleiche Bezugszeichen kennzeichnen gleiche oder ähnliche Merkmale der gezeigten Ausführungsbeispiele. Es zeigen:
- Figur 1A: in einer perspektivischen Darstellung der Anlage für biotechnologische Anwendungen zum Aufnehmen eines Einwegbehälters;
- Figur 1B: in einer Draufsicht die in Fig. 1A gezeigte Anlage zum Aufnehmen eines Einwegbehälters;
- Figur 2A: in einer perspektivischen Darstellung einen Sondenhalter für Sonden, die bereichsweise in einen Einwegbehälter eingreifen;
- Figur 2B: in einer Draufsicht den in Fig. 2A gezeigten Sondenhalter für die Sonden, die bereichsweise in einen Einwegbehälter eingreifen;
- Figur 3A: in einer Seitenansicht den in Fig. 2A gezeigten Sondenhalter für Sonden, die bereichsweise in einen Einwegbehälter eingreifen;
- Figur 3B: in einer seitlichen Schnittdarstellung den in Fig. 2A gezeigten Sondenhalter für Sonden, die bereichsweise in einen Einwegbehälter eingreifen;
- Figur 4A: in einer Seitenansicht eine Halterungsbasis eines Sondenhalters;
- Figur 4B: in einer Frontalansicht ein zweites, bewegliches Bauteil der Halterungsbasis eines Sondenhalters;
- Figur 5A: in einer ersten perspektivischen Darstellung ein Sondenanordenmittel eines Sondenhalters; und
- Figur 5B: in einer zweiten perspektivischen Darstellung das in Fig. 5A gezeigte Sondenanordenmittel eines Sondenhalters.

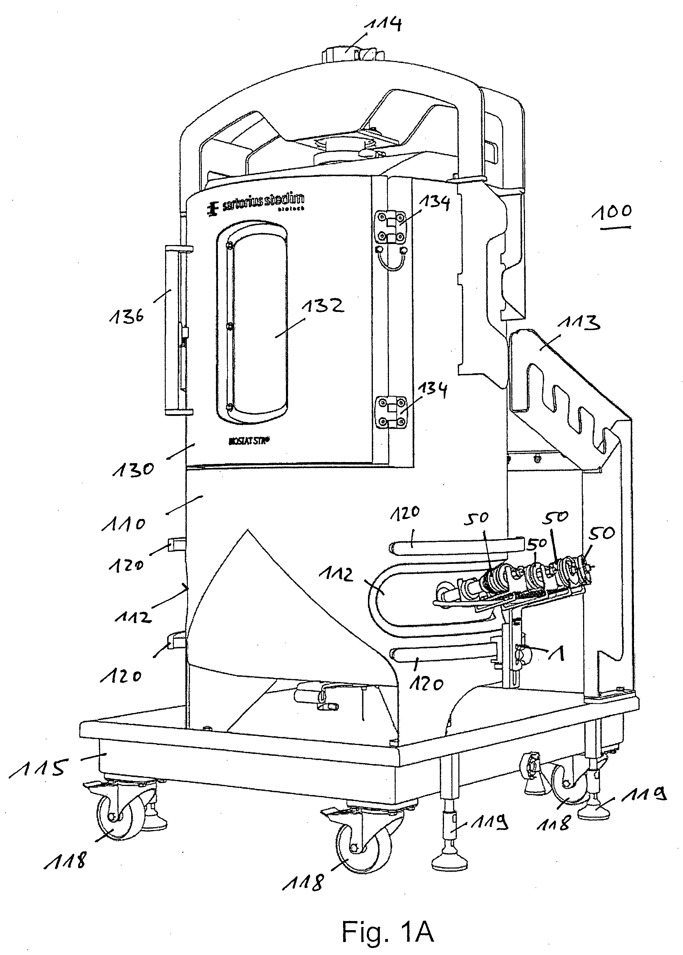

**Figur 1A** zeigt in einer perspektivischen Darstellung eine Anlage 100 zum Aufnehmen eines Einwegbehälters. Die Anlage 100 ist als eine Anlage für biotechnologische Anwendungen ausgebildet und weist einen Aufnahmebehälter 110 auf, der insbesondere im Wesentlichen die Form eines (insbesondere im Wesentlichen vertikal angeordneten) Zylinders aufweist, d.h. dessen Zylinderachse im Wesentlichen vertikal angeordnet ist. Der Aufnahmebehälter 110 weist einen Behälterinnenraum auf, in den ein Einwegbehälter zumindest teilweise eingebracht werden kann, der zum Beispiel ein biologisches Medium beinhalten kann. Das biologische Medium im Einwegbehälter wird im Behälterinnenraum des Aufnahmebehälters 110 über einen vorbestimmbaren Zeitraum und/oder steril gelagert. Während sich der Einwegbehälter mit dem biologischen Medium im Inneren des Aufnahmebehälters 110 befindet, können sich unterschiedliche Reaktionen mit oder an dem biologischen Medium ereignen. Somit kann die Anlage 100 auch als Bioreaktor und/oder Pallettank ausgebildet sein.

Bei der Anlage 100 kann es sich insbesondere um Single-Use-Anlagen für biotechnologische Anwendungen, Crossflow-Anlagen, Filtrationsanlagen, Bioreaktorbehälter, Biogasanlagen, Mischer bzw. Mischsysteme, Schüttler, Einfrier- und Auftaubehälter, Vorrichtungen für die Behandlung von Fluiden und/oder ähnliche Anlagen handeln. Die Anlage für biotechnologische Anwendungen (z.B. Bioreaktoren) kann insbesondere Behälter umfassen, in denen speziell herangezüchtete Mikroorganismen und/oder Zellen unter möglichst optimalen kontrollierten (insbesondere sterilen) Bedingungen in einem Nährmedium kultiviert werden, um entweder die Zellen selbst, Teile von ihnen und/oder eines ihrer Stoffwechselprodukte zu gewinnen. Hierzu werden ein oder mehrere Zu- und/oder Ableitungen für die jeweiligen einzelnen Produkte bzw. Stoffe benötigt. Im Speziellen können in den Bioreaktoren Anlagen feste (Biomasse), flüssige (Nährmedium) und/oder gasförmige (z.B. Luft, Sauerstoff, Kohlendioxid, Stickstoff) Phasen verarbeitet werden. Um optimale Bedingungen zu gewährleisten, werden üblicherweise ein oder mehrere (verschiedene) Parameter im Innenraum der Anlage mit Hilfe von Sensoren und/oder Sonden 50 gemessen bzw. überwacht, die in den Innenraum der Anlage hineinragen. Mögliche zu messende Parameter sind beispielsweise der pH-Wert, der 02-Wert und die Temperatur des in der Anlage enthaltenen Mediums. Sollten Parameter von vordefinierten Optimalwerten abweichen, können mittels geeigneter Maßnahmen die Abweichungen korrigiert werden. Anlagen können zur mehrmaligen Nutzung oder als Einweganlage bzw. -bioreaktor ausgelegt sein. Die Anlage 100 kann als eine sterile Einheit an den Benutzer ausgeliefert werden.

Zur Beobachtung und/oder Überwachung des biologischen Mediums sind in den Seitenwänden ein oder mehrere Sichtfenster ausgebildet, durch das bzw. die von außen durch die Aufnahmebehälterwand hindurch in den Behälterinnenraum des Aufnahmebehälters 110 hineingeblickt werden kann, um das biologische Medium zu beobachten, und/oder ein oder mehrere Sonden durch den Aufnahmebehälter 110 hindurch in den Einwegbehälter zumindest teilweise hineinzuragen bzw. dessen Inhalt zu untersuchen und/oder zu kontrollieren. Die Anlage 100 weist dazu in einem unteren Bereich (z.B. im unteren Drittel) ein oder mehrere (z.B. zwei) Bodenfenster 112 und/oder ein Türfenster 132 auf. Die Bodenfenster 112 sind insbesondere im Wesentlichen in Form eines länglichen Ovals ausgebildet, dessen lange Ovalachse im Wesentlichen horizontal entlang der (insbesondere gekrümmten) Zylinderaußenwand des Aufnahmebehälters 110 ausgerichtet ist. Das Türfenster 132 ist im Wesentlichen in Form eines länglichen Rechtecks ausgebildet, wobei deren längere Seiten im Wesentlichen vertikal ausgerichtet sind und in der Mitte einer Einblatttür 130 in der Behälterwand des Aufnahmebehälters 110 ausgebildet sind.

Die Einblatttür 130 ist im Wesentlichen im oberen Bereich (z.B. in den oberen zwei Dritteln) des Aufnahmebehälters 110 ausgebildet, während der untere Bereich (z.B. das untere Drittel) des Aufnahmebehälters 110 im Wesentlichen in Form einer steifen Bodenschale ausgebildet ist, die selber nicht öffenbar ausgebildet ist. Die Einblatttür 130 ist um Türscharniere 134 drehbar und somit öffenbar. Dabei kann ein Türgriff 136 vorgesehen sein zum Öffnen der Einblatttür 130. Ist die Einblatttür 130 geöffnet, so ist in dem Aufnahmebehälter 110 an einer lateralen Position eine Türöffnung ausgebildet, durch die ein Zugriff auf den Behälterinnenraum des Aufnahmebehälters 110 ermöglicht wird. Durch die Türöffnung kann zum Beispiel der Einwegbehälter von einer seitlichen, lateralen Richtung her, also im Wesentlichen in einer horizontalen Bewegungsrichtung, in den Behälterinnenraum des Aufnahmebehälters 110 eingeführt werden.

Die Anlage 100 ist auf Rollen 118 verschiebbar bzw. rollbar gelagert, auf denen die Anlage durch einen Raum geschoben werden kann. Zusätzlich zu den Rollen 118 kann die Anlage 100 am unteren Ende Fixierfüße 119 aufweisen, die zum Fixieren und korrektem Ausrichten der Anlage 100 auf unebenen Böden dienen.

Der Aufnahmebehälter 110 kann nach oben offen ausgebildet sein. An Stelle eines oberen Zylinderdeckels weist der Aufnahmebehälter 110 eine Rühröffnung auf. Oberhalb des nach oben offenen Aufnahmebehälters 110 ist eine Rührvorrichtung 114 ausgebildet, über die ein Rührstab durch die Rühröffnung hindurch mit dem Einwegbehälter so verbunden werden kann, dass das Innere des Einwegbehälters durchmischt werden kann. Der Rührstab kann im Inneren des Einwegbehälters angeordnet sein und über eine Kopplung bzw. Kupplung mit der Rührvorrichtung 114 verbunden werden. Die Rührvorrichtung 114 ist zentral über dem Aufnahmebehälter 110 ausgebildet und wird von einer Trägerbrücke getragen, die auf einem oberen Rand des Aufnahmebehälters 110 an einander gegenüberliegenden Seitenwänden des Aufnahmebehälters 110 aufliegt.

Seitlich an der Anlage 100 können Kabelführungen 113 angeordnet sein, in denen Anschlusskabel für die Rührvorrichtung 114 und/oder Sensoren/Sonden geführt sein können.

In den Behälterinnenraum des Aufnahmebehälters 110 und zugleich auch in das Innere des Einwegbehälters kann ein biologisches Medium eingefüllt werden. Der Einwegbehälter kann nach Verwendung zum Beispiel über ein unterhalb der Anlage 100 angeordneten Auslass entleert und anschließend vollständig entsorgt werden. Durch Verwendung des Einwegbehälters kann eine Reinigung der Anlage 100 eingespart werden, bzw. wesentlich schneller erfolgen.

Ein unterhalb des Aufnahmebehälters 110 angeordneter Auffangbehälter 115 dient als Auffangelement, falls aus der Anlage 100 z.B. durch ein Leck im Einwegbeutel biologisches Medium austreten sollte.

Benachbart und/oder angrenzend zu den Bodenfenstern 112 sind Systemschienen 120 angeordnet. Hierbei sind die Systemschienen 120 so angeordnet, dass sie im Wesentlichen parallel zur einer Längsrichtung des jeweiligen Bodenfensters 112 entlang einer Außenfläche der Anlage 100 verlaufen. In der gezeigten Ausführungsform weist die Anlage 100 sowohl vertikal oberhalb als auch vertikal unterhalb der Bodenfenster 112 jeweils eine Systemschiene 120 auf, die der Außenkrümmung des Aufnahmebehälters 110 folgt und jeweils in einer im Wesentlichen horizontalen Ebene angeordnet ist.

An einer der Systemschienen 120, im Ausführungsbeispiel an einer unterhalb des Bodenfensters 112 angeordneten Systemschiene 120, ist ein Sondenhalter 1 angeordnet. Der Sondenhalter 1 trägt mehrere Sonden 50, die so angeordnet sind, dass sie durch das Bodenfenster 112 hindurch weisen. Der Sondenhalter 1 ist dazu ausgebildet, jede der Sonden 50 tragend in einer jeweils eigenen Sollposition anzuordnen und auszurichten.

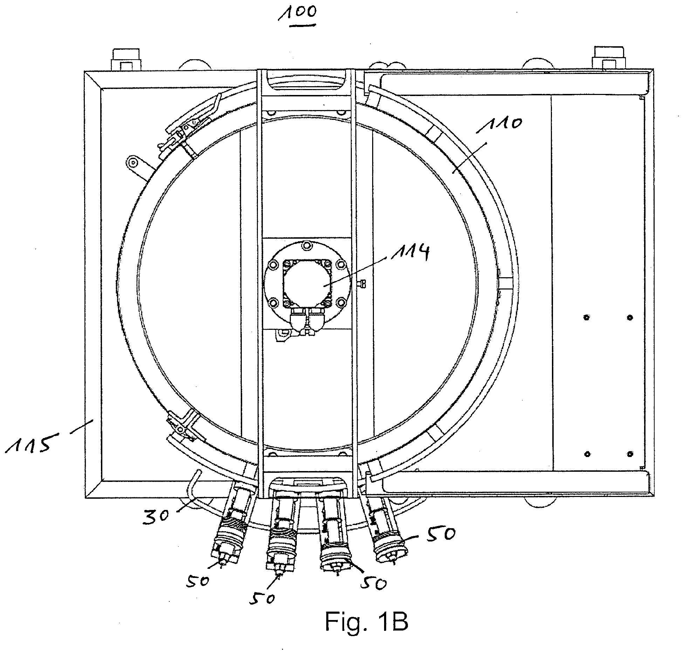

**Figur 1B** zeigt in einer Draufsicht auf die in Fig. 1A gezeigte Anlage 100 zum Aufnehmen eines Einwegbehälters. Der Sondenhalter 1 ist zur Aufnahme von vier Sonden 50 ausgebildet. Jede der Sonden 50 ist dabei so ausgerichtet, dass ein Sondenkopf jeder der Sonden 50 (in der gezeigten Draufsicht) etwa in Richtung zum Mittelpunkt des Aufnahmebehälters 110 hin weist. Hierbei liegt der Mittelpunkt des Aufnahmebehälters 110 auf einer vertikal angeordneten Zylinderachse des Aufnahmebehälters 110.

Die vier Sonden 50 sind auf einer Befestigungsstange 30 des Sondenhalters 1 angeordnet und jeweils etwa gleich weit voneinander beabstandet. Die Befestigungsstange 30 ist gebogen ausgebildet und folgt mit ihrer Biegung der konvexen Außenwölbung des Aufnahmebehälters 110 der Anlage 100, so dass die Befestigungsstange 30 im Wesentlichen bereichsweise parallel zur Außenfläche des Aufnahmebehälters 110 ausgebildet ist. Wie auch in Fig. 1A gezeigt, ist die Befestigungsstange 30 im Wesentlichen in einer horizontalen Ebene angeordnet. Die Befestigungsstange 30 kann als Teil einer Halterungsbasis 10 ausgebildet sein, die nachfolgend näher beschreiben ist.

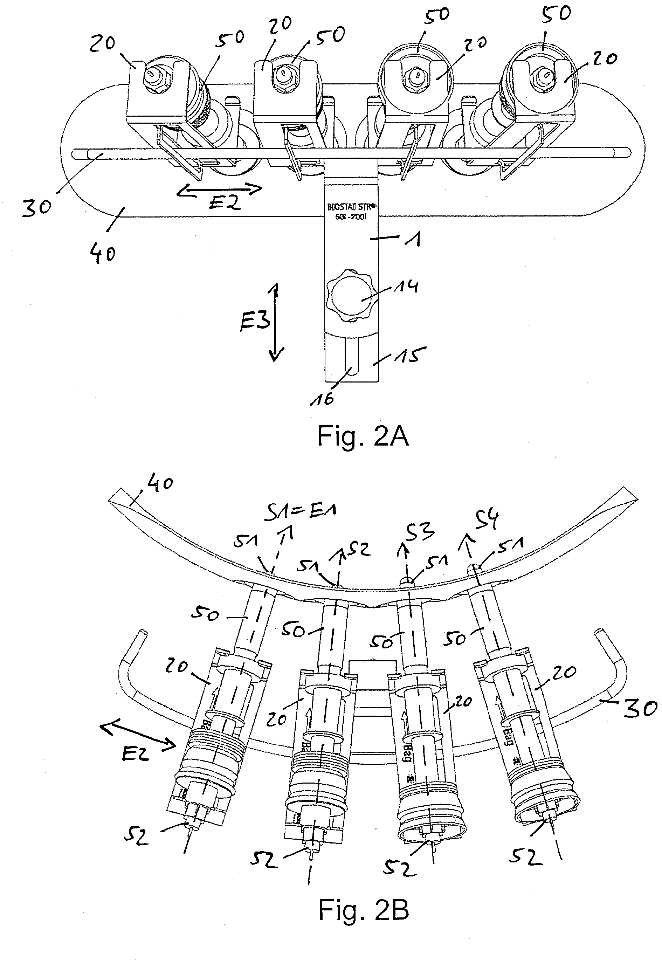

**Figuren 2A und 2B** zeigen in einer perspektivischen Darstellung und in einer Draufsicht den Sondenhalter 1 für die ein oder mehrere Sonden 50, die (insbesondere durch das Bodenfenster 112 hindurch) zumindest teilweise in einen Einwegbehälter eingreifen. In den beiden Figuren ebenfalls gezeigt ist ein Ausschnitt einer Behälterwand 40 des Einwegbehälters, der insbesondere durch das Bodenfenster 112 von außen zugänglich ist. Die Behälterwand 40 kann flexibel ausgebildet sein, z.B. aus einem Kunststoff. Deswegen ist die Position der Behälterwand 40 nicht starr fixiert, sondern sie kann sich im Laufe der Zeit ändern. Insbesondere beim Befüllen des Behälters mit dem Medium oder über einen längeren Lagerungszeitraum kann sich die Behälterwand 40 z.B. ausdehnen.

Die Sonden 50 sind in einer Sollposition angeordnet, in der sie vom Sondenhalter 1 getragen werden. In dieser Sollposition ist jeweils ein Sondenkopf 51 jeder der Sonden 50 so angeordnet, dass er durch das Bodenfenster 112 hindurch und durch die Behälterwand 40 hindurch zumindest teilweise ins Innere des Einwegbehälters eingreift und/oder hineinragt, wobei insbesondere eine Sterilität gewährleistet ist. Dabei steht jeder der Sondenköpfe 51 insbesondere in unmittelbarem Berührkontakt mit dem z.B. biologischen Medium, dass ins Innere des Einwegbehälters eingefüllt ist. Die Sondenköpfe 51 können dabei einmalig oder wiederholt bzw. über einen längeren Zeitraum hinweg Messungen und/oder Untersuchungen an dem Medium vornehmen, insbesondere zur Messung des pH-Werts des Mediums, und/oder zur Messung einer bestimmten Konzentration im Medium, zur Messung einer Lichtdurchlässigkeit, zur Bestimmung der Biomasse usw.

Eine oder mehrere der Sonden 50 können auch als Spektroskop ausgebildet sein. Jede Sonde 50 kann zur Messung und/oder Bestimmung eines anderen Messwerts ausgebildet sein. Die Sonden 50 können sich somit voneinander unterscheiden. Die Sonden 50 können zumindest teilweise als Single-Use Sensoren ausgebildet sein, und/oder zumindest teilweise als wiederverwendbare Sensoren.

Jede der Sonden 50 ist im Wesentlichen länglich ausgebildet und erstreckt sich von einem Befestigungsende 52, welches an einem dem Sondenkopf 51 abgewandten Ende der jeweiligen Sonde 50 ausgebildet ist, bis hin zum jeweiligen Sondenkopf 51. Jede der Sonden 50 kann an einem Sondenbefestigungsbereich, der angrenzend und/oder benachbart zum Befestigungsende 52 ausgebildet sein kann, in jeweils einem Sondenanordenmittel 20 angeordnet, befestigt und/oder aufgenommen sein.

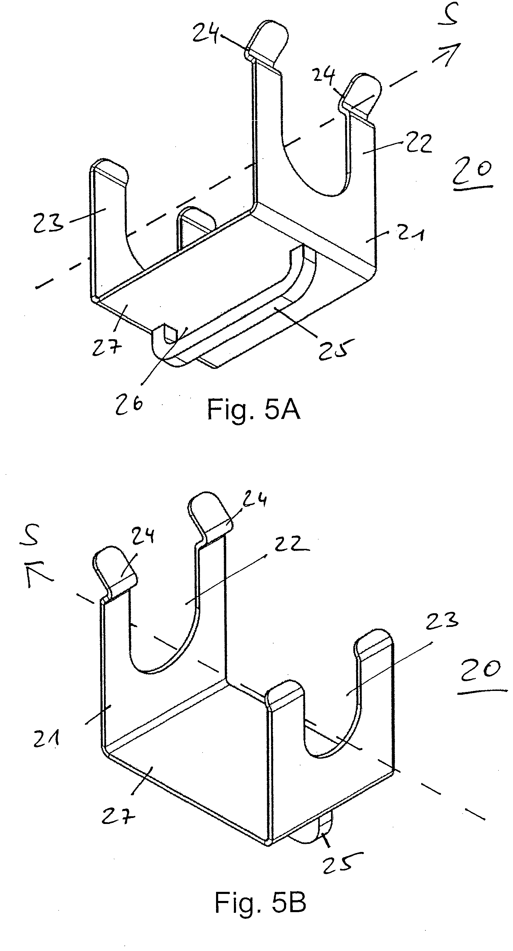

Der Sondenhalter 1 weist ein oder mehrere Sondenanordenmittel 20, insbesondere so viele Sondenanordenmittel 20 auf, wie er Sonden 50 halten und/oder aufnehmen kann, im gezeigten Ausführungsbeispiel also vier Sondenanordenmittel 20. Die Sondenanordenmittel 20 sind nachfolgend näher mit Bezug auf die Figuren 5A und 5B beschrieben und gezeigt.

In einer Betriebsposition ist jede der Sonden 50 so in ihrer Sollposition angeordnet, dass sie in ihre jeweilige Sondenerstreckungsrichtung S1 bis S4 weist und/oder ausgerichtet ist. Dies bedeutet für jede Sonde 50, dass eine geradlinige Verbindung von ihrem Befestigungsende 52 zu ihrem Sondenkopf 51 im Wesentlichen mit der ihr zugeordneten Sondenerstreckungsrichtung S1 bis S4 zusammenfällt. Mit anderen Worten können die Längsachsen der Sonden 50 jeweils in Sondenerstreckungsrichtung S1, S2, S3 und/oder S4 ausgerichtet sein.

Hierbei kann jede der Sondenerstreckungsrichtungen S1 bis S4 ins Innere des Aufnahmebehälters 110 weisen, insbesondere hin zu einem Mittelpunkt und/oder einer z.B. vertikalen Zylinderachse des Aufnahmebehälters 110.

In der in Fig. 2B gezeigten Draufsicht von vertikal oben auf den Sondenhalter 1 sind die Sondenerstreckungsrichtungen S1 bis S4 so angeordnet, dass sie die Behälterwand 40 jeweils in etwa im Lot durchstoßen.

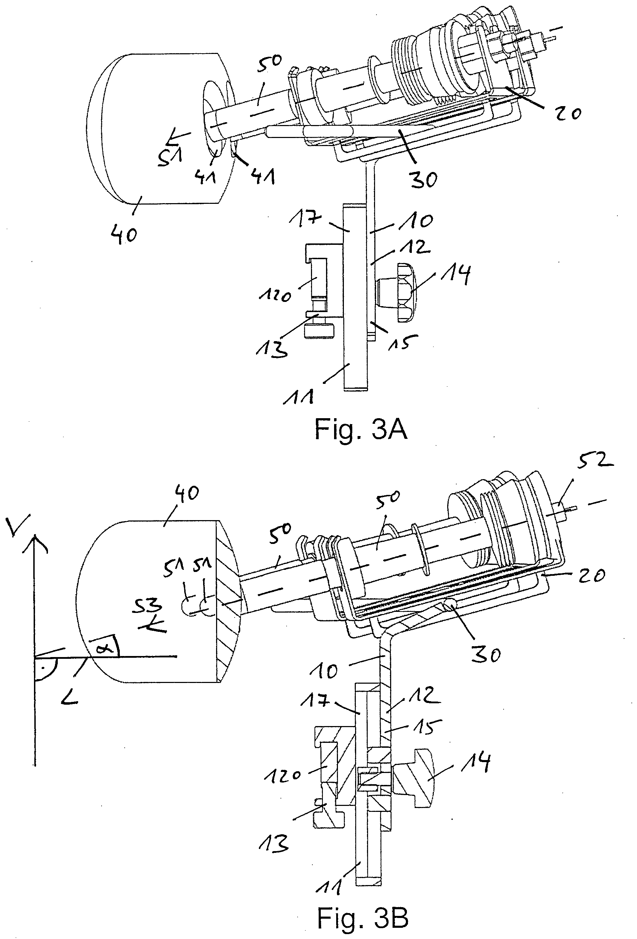

**Figuren 3A und 3B** zeigen den Sondenhalter 1 in einer Seitenansicht und in einer seitlichen Schnittdarstellung.

Aus dieser seitlichen Ansicht auf den Sondenhalter 1 in eine im Wesentlichen horizontale Richtung ist gezeigt, dass die Sondenerstreckungsrichtungen S1 bis S4 so angeordnet sind, dass sie die Behälterwand 40 jeweils nicht im Lot durchstoßen. In Fig. 3B ist beispielhaft die dritte Sondenerstreckungsrichtung S3 gezeigt, die die Behälterwand 40 in etwa unter einem Neigungswinkel α zum Lot L auf die Behälterwand 40 schneidet. Dieser Neigungswinkel α kann z.B. von etwa 10° bis etwa 20° betragen und kann abhängig vom Sondentyp der jeweiligen Sonde 50 sein. Allgemein ist es aufgrund der Funktionsweise und der Bauweise der Sonden 50 üblich, diese unter einem gewissen Neigungswinkel α zum Lot auf die der Sonde 50 zugewandte Außenfläche des Einwegbehälters auszurichten.

Im gezeigten Ausführungsbeispiel ist der Abschnitt der Behälterwand 40, den der Sondenkopf 51 durchstößt, im Wesentlichen parallel zu einer Vertikalen V ausgerichtet. Deswegen ist das Lot L auf diesen Abschnitt der Behälterwand 40 in etwa parallel zur Horizontalen.

An der Behälterwand 40 können Andockvorrichtungen 41 vorgesehen sein, die zum Andocken und Einführen der Sondenköpfe 51 ausgebildet sind. Die Andockvorrichtung 41 bildet einen Sensorport für die Sonde 50, die mit ihrem Sondenkopf 51 an der Behälterwand 40 des Einwegbehälters befestigt und/oder fixiert werden kann. Hierbei kann insbesondere eine Sterilverbindung zwischen dem Sondenkopf und dem Innenraum des Einwegbehälters hergestellt werden. Hierbei kann die Sonde 50 fest an dem Einwegbehälter befestigt werden, wobei sie vom Sondenhalter 1 unterstützend getragen wird.

Zur Anordnung und Ausrichtung der Sonden 50 in ihrer jeweiligen Sondenerstreckungsrichtung S1 bis S4 weist der Sondenhalter 1 die Halterungsbasis 10 auf, die an der Systemschiene 120 der Anlage 100 befestigt ist, sowie die Sondenanordenmittel 20.

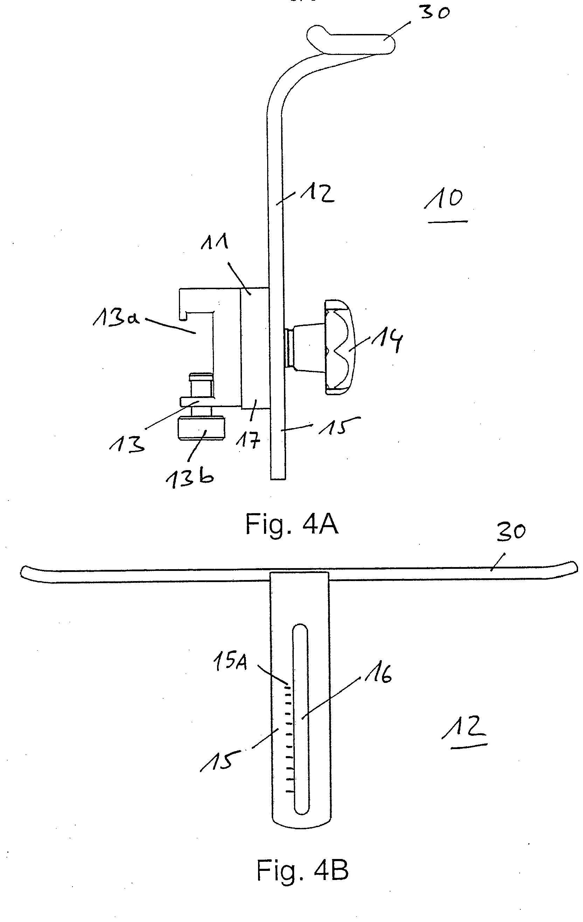

**Figur 4A** zeigt in einer Seitenansicht aus einer im Wesentlichen horizontalen Richtung die Halterungsbasis 10 des Sondenhalters 1. Die Halterungsbasis 10 ist insbesondere im Wesentlichen zweiteilig ausgebildet. Ein erstes Bauteil 11 der Halterungsbasis 10 ist als ein anlagenfestes Bauteil ausgebildet und fest bzw. ortsfest an die Anlage anbringbar. Ein zweites Bauteil 12 der Halterungsbasis 10 ist als ein bewegliches Bauteil ausgebildet, insbesondere als ein relativ zur Anlage 100 bewegliches Bauteil.

Das erste, anlagenfeste Bauteil 11 umfasst insbesondere ein Anbringmittel 13 zum Anbringen des Sondenhalters 1 an der Anlage 100. Das Anbringmittel 13 kann zum Befestigen des Sondenhalters 1 an einer der Systemschienen 120 ausgebildet sein. Das Anbringmittel 13 kann z.B. eine Aussparung 13a zum An- bzw. Aufbringen des Anbringmittels 13 auf die Systemschiene 120 aufweisen und ein Schraubmittel 13b (als ein besonderes lösbares Fixiermittel). Das Schraubmittel 13 kann ein in die Aussparung 13 einschraubbares Ende aufweisen, mit dem der Sondenhalter 1 an der in die Aussparung 13a angeordneten Systemschiene 120 (insbesondere lösbar) befestigt werden kann.

Das erste, anlagenfeste Bauteil 11 kann weiterhin einen Block 17 umfassen, der starr mit dem Anbringmittel 13 und somit der Anlage 100 verbunden ist. In den Block 17 kann ein Stellrad 14 eingeschraubt sein. Zwischen dem Stellrad 14 und dem Block 17 ist eine Schiene 15 des zweiten, beweglichen Bauteils 12 angeordnet und festgeschraubt.

**Figur 4B** zeigt in einer Frontalansicht das zweite, bewegliche Bauteil 12 der Halterungsbasis 10 des Sondenhalters 1. Das zweite, bewegliche Bauteil 12 weist die Schiene 15 auf, in der eine Nut bzw. Langloch 16 ausgebildet ist. An einem z.B. oberen Ende der Schiene 15 ist die Befestigungsstange 30 ausgebildet. Auf bzw. an der Schiene 15 kann eine Skala bzw. Einstellhilfe 15A angebracht sein, um eine Lage des beweglichen Bauteils 12 einfacher einstellen zu können. Eine Skala und/oder Einstellhilfe kann in einer alternativen Ausführung auch an den weiteren Bauteilen des Sondenhalters 1 wie z.B. am Sondenanordenmittel 20 angeordnet sein.

Die Nut 16 ist etwa geradlinig ausgebildet und verläuft entlang einer Längsrichtung der Schiene 15. Im gezeigten Ausführungsbeispiel ist die Nut 16 genauso wie die Schiene 15 in einer im Wesentlichen vertikalen Richtung angeordnet. Allgemein kann die Nut 16 und die Schiene 15 im Wesentlichen parallel zu einer benachbarten Außenoberfläche des Aufnahmebehälters 110 ausgebildet sein, insbesondere parallel zur ungekrümmten Verlaufsrichtung der benachbarten Außenoberfläche des Aufnahmebehälters 110.

In einer Betriebsposition greift das Stellrad 14 und/oder eine Schraube des Stellrads 14 in die Nut 16 ein (siehe Fig. 4A und 2A). Durch Betätigung des Stellrads 14 kann die Schiene 15 entweder an das erste, anlagenfeste Bauteil 11 der Halterungsbasis 10 festgeschraubt werden, oder davon losgeschraubt werden. Im losgeschraubten Zustand ist das zweite, bewegliche Bauteil 12 relativ zum ersten, anlagenfesten Bauteil 11 verschiebbar und/oder beweglich. Auf diese Weise kann die Position des zweiten, beweglichen Bauteils 12 relativ zur Anlage 100 und insbesondere relativ zur im Bodenfenster 112 angeordneten Andockvorrichtung 41 des Einwegbehälters eingestellt werden.

Benachbart zur Nut 16 kann auf der Schiene 15 eine Skala vorgesehen sein, mit der die Position des zweiten, beweglichen Bauteils 12 relativ zum ersten, anlagenfesten Bauteil 11 eingestellt werden kann.

Alternativ oder zusätzlich kann der Block 17 eine Rippe oder Feder aufweisen, die in die Nut 16 eingreift und die Ausrichtung der beiden Bauteile 11 und 12 der Halterungsbasis 10 zueinander verbessert. Die Rippe und/oder Feder kann im Wesentlichen parallel zur Nut 16 ausgebildet sein, z.B. in etwa in die voranstehend genannte ungekrümmte Verlaufsrichtung der benachbarten Außenoberfläche des Aufnahmebehälters 110.

Die Befestigungsstange 30 kann an ihren Enden gekrümmt ausgebildet sein und/oder Schlaufen aufweisen, z.B. zur Aufnahme von Kabeln der Sonden 50. Alternativ zu den in den Figuren gezeigten Ausführungsform kann die Befestigungsstange 30 auch im Wesentlichen gestreckt, also ungekrümmt, ausgebildet sein. Weiterhin kann ein Befestigungsstange auch lediglich einseitig an der Schiene 15 ausgebildet sein. Die Befestigungsstange könnte auch in Draufsicht unter einem Winkel geneigt zur Außenfläche der der Sonde 50 zugewandten Außenfläche der Behälterwand 40 ausgebildet sein. Zudem können an der Schiene 15 auch mehrere Befestigungsstangen z.B. zueinander versetzt angeordnet sein.

Weiterhin kann an der Halterungsbasis 10 ein Außenschutzblech zum Schutz der ausgerichteten Sonden 50 gegenüber unerwünschten Außeneinflüssen wie z.B. Stößen ausgebildet sein. An dem Sondenhalter 1 kann eine Sicherung ausgebildet sein, die die Sonden 50 z.B. gegen ein Herunterfallen sichert.

**Figuren 5A und 5B** zeigen in einer ersten und zweiten perspektivischen Darstellung eines der Sondenanordenmittel 20 des Sondenhalters 1. Das Sondenanordenmittel 20 weist eine Sondenaufnahme 21 als einen Bereich auf, der zum Aufnehmen von z.B. genau einer der Sonden 50 ausgebildet ist. Die Sondenaufnahme 21 kann dabei z.B. etwa klammerförmig und/oder etwa U-förmig ausgebildet sein.

Die Sondenaufnahme 21 weist einen ersten Haltebereich 22 und einen zweiten Haltebereich 23 auf. Die beiden Haltebereiche 22, 23 sind zum mechanischen Kontaktieren, Festhalten und/oder Aufnehmen zweier in Sondenerstreckungsrichtung S voneinander beabstandeter Haltepunkte der Sonde 50 ausgebildet. So können die beiden Haltebereiche 22, 23 z.B. jeweils eine Aussparung mit einem Innendurchmesser und/oder einer Innenkontur aufweisen, der/die komplementär zu einem Außendurchmesser und/oder einer Außenkontur der Sonde 50 am jeweils zugeordneten Haltepunkt der Sonde 50 ausgebildet ist. Die Aussparungen der Haltebereiche 22, 23 können durch Fingerenden und/oder Klammerenden der Sondenaufnahme 21 definiert werden. Durch die Ausbildung der Haltebereiche 22, 23 wird die Position bestimmt und/oder definiert, die die Sonde 50 in dem Sondenanordenmittel 20 einnimmt.

Die Sondenaufnahme 21 kann weiterhin Rastmittel 24 aufweisen, insbesondere angrenzend an, benachbart zu und/oder in zumindest einem der Haltebereiche 22, 23. Die Rastmittel 24 ermöglichen ein Verrasten der Sonde 50 in der Sondenaufnahme 21. Insbesondere können die Rastmittel 24 an den Fingerenden und/oder Klammerenden zumindest eines der Haltebereiche 22 und/oder 23 ausgebildet sein.

Die beiden Haltebereiche 22, 23 sind über einen Mittelbereich 27 voneinander beabstandet. Der Mittelbereich 27 kann im Wesentlichen flächig und/oder als ein Blech ausgebildet sein. Die Sondenerstreckungsrichtung S kann in etwa parallel zu einer Ausbreitungsebene des Mittelbereichs 27 ausgebildet sein. Die Länge des Mittelbereichs 27 in Sondenerstreckungsrichtung S kann dabei kürzer sein als die Länge der Sonde 50.

Eine Sonde 50 kann mit ihrem an ihrem Befestigungsende 52 angrenzenden Bereich in den beiden Haltebereichen 22, 23 so angeordnet werden, dass der Sondenkopf 51 aus dem Sondenanordenmittel 20 herausragt, insbesondere in Sondenerstreckungsrichtung S.

Die Sonden 50 können eine Faltenbalg aufweisen, mit dem die Sonden 50 in die beiden Haltebereiche 22, 23 des Sondenhalters 1 eingebracht und/oder eingeklemmt werden können. Insbesondere können die Sonden 50 mit z.B. ausgezogenem Faltenbalg während eines Abdichtens, Ausrichtens und/oder Einstellens in dem Sondenanordenmittel 20 gehalten und getragen werden.

An einer Seite der Sondenaufnahme 21, die in einer Betriebsposition von der Sonde 50 abgewandt ist, ist eine Klemmschiene 25 ausgebildet. Die Klemmschiene 25 kann im Wesentlichen parallel zur Sondenerstreckungsrichtung S angeordnet sein und an zumindest einer, bevorzugt zumindest zwei Stelle(n) am Mittelbereich 27 befestigt sein.

Zwischen der Klemmschiene 25 und dem Mittelbereich 27 ist eine Stangenaufnahme 26 als ein Freiraum ausgebildet. Die Stangenaufnahme 26 dient zur Aufnahme der Befestigungsstange 30. Hierbei kann der Freiraum zwischen der Klemmschiene 25 und dem Mittelbereich 27 im Wesentlichen so groß wie der Durchmesser der Befestigungsstange 30 ausgebildet sein. Insbesondere kann die Stangenaufnahme 26 etwas kleiner als der Durchmesser der Befestigungsstange 30 ausgebildet sein, z.B. von etwa 0,1% bis etwa 5% kleiner als der Durchmesser der Befestigungsstange 30. Dadurch wird es ermöglicht, das Sondenanordenmittel 20 in einem Klemmsitz auf der Befestigungsstange 30 anzuordnen, wie dies z.B. in Fig. 2A gezeigt ist.

Alternativ kann die Stangenaufnahme 26 auch zumindest so groß wie der Durchmesser der Befestigungsstange 30 ausgebildet sein, z.B. von etwa 0% bis etwa 50% größer, bevorzugt von etwa 5% größer bis etwa 25% größer, als der Durchmesser der Befestigungsstange 30. Dadurch wird es ermöglicht, dass das Sondenanordenmittel 20 besonderes einfach in Sondenerstreckungsrichtung S verschoben werden kann, insbesondere bei Krafteinwirkung durch ein Ausdehnen und/oder Zusammenziehen der Behälterwand 40. Hierbei kann ein eckig ausgebildeter Querschnitt der Befestigungsstange 30 ein Verkippen und/oder Verschwenken des Sondenanordenmittels 20 um die Befestigungsstange 30 herum verhindern oder reduzieren. Ein solch eckig ausgebildeter Querschnitt der Befestigungsstange 30 kann in diesem Fall auf einen z.B. eckig ausgebildeten Querschnitt der Klemmschiene 25 abgestimmt sein.

Die einzelnen Sondenanordenmittel 20 können auf die Befestigungsstange 30 in eine im Wesentlichen horizontale Richtung aufgebracht, eingefädelt, und/oder aufgeschoben werden.

Der Querschnitt der Klemmschiene 25 und/oder der Befestigungsstange 30 kann zumindest bereichsweise rund, eckig und/oder quaderförmig ausgebildet sein. Die beiden Querschnitte können aufeinander abgestimmt sein, um einen möglichst guten Halt der beiden Bauteile aneinander zu ermöglichen.

Die genaue Position der Sondenanordenmittel 20 auf der Befestigungsstange 30 kann mit Hilfe von Ringen und/oder Klemmen auf der Befestigungsstange 30 gegen ein Verrutschen in Stangenerstreckungsrichtung und/oder ein Verschwenken um die Befestigungsstange 30, etc. gesichert sein.

Diese Art der Befestigung des Sondenanordenmittels 20 an der Befestigungsstange 30 ermöglicht ein Einstellen der Position der Sonde 50 in eine erste Einstellrichtung, nämlich in Sondenerstreckungsrichtung S. So kann das Sondenanordenmittel 20 in und gegen die Sondenerstreckungsrichtung S verschoben werden, wenn die Befestigungsstange 30 in der Stangenaufnahme 26 z.B. im Klemmsitz angeordnet ist und entlang der Stangenaufnahme 26 verschoben wird.

Im gezeigten Ausführungsbeispiel ist die erste Einstellrichtung, also die Sondenerstreckungsrichtung S, um den Neigungswinkel α zur Horizontalen geneigt. Allgemein kann die erste Einstellrichtung um einen Neigungswinkel α zum Lot auf die der Sonde 50 zugewandten Außenflächen der Behälterwand 40 ausgerichtet sein.

Zudem kann das Sondenanordenmittel 20 in Stangenerstreckungsrichtung der Befestigungsstange 30 verschoben werden, was eine zweite Einstellrichtung der Position der Sonde ermöglicht. Die Stangenerstreckungsrichtung entspricht dem Verlauf der z.B. gebogen ausgebildeten Befestigungsstange 30, die mit ihrer Biegung der konvexen Außenwölbung des benachbarten Aufnahmebehälters 110 der Anlage 100 folgen kann, so dass die Befestigungsstange 30 im Wesentlichen bereichsweise parallel zur benachbarten Außenfläche des Aufnahmebehälters 110 ausgebildet ist. Die zweite Einstellrichtung kann somit insbesondere einer gekrümmten Außenfläche der Anlage 100 folgen, und zwar entlang der gekrümmten Verlaufsrichtung und/oder Krümmungsrichtung der benachbarten Außenoberfläche.

Im gezeigten Ausführungsbeispiel erstreckt sich die zweite Einstellrichtung in einer horizontalen Ebene. Allgemein kann die zweite Einstellrichtung etwa parallel zu einer ersten Verlaufsrichtung der benachbarten Außenfläche der Anlage 100 und/oder des Einwegbehälters ausgebildet sein.

Durch die im Zusammenhang mit den Figuren 4A und 4B beschriebe zweiteilig ausgebildete Halterungsbasis 10 kann eine Position der Sonden 50 relativ zur Anlage 100 und/oder relativ zum Einwegbehälter in eine dritte Einstellrichtung eingestellt werden. Diese dritte Einstellrichtung kann parallel zu einer benachbarten Außenoberfläche des Aufnahmebehälters 110 ausgebildet sein, insbesondere parallel zur ungekrümmten Verlaufsrichtung der benachbarten Außenoberfläche des Aufnahmebehälters 110.

Im Ausführungsbeispiel ist die dritte Einstellrichtung eine im Wesentlichen vertikale Richtung. Allgemein kann die dritte Einstellrichtung etwa parallel zu einer zweiten Verlaufsrichtung der benachbarten Außenfläche der Anlage 100 und/oder des Einwegbehälters ausgebildet sein. Die zweite Verlaufsrichtung kann unterschiedlich von der ersten Verlaufsrichtung der benachbarten Außenfläche ausgebildet sein, insbesondere können die beiden Verlaufsrichtungen im Wesentlichen senkrecht zueinander ausgebildet sein. Hierbei muss keine der Verlaufsrichtungen einer Krümmung des Aufnahmebehälters folgen, z.B. bei einem Aufnahmebehälter mit einer ebenen Wandung.

Für eine erste der Sonden 50 sind die drei Einstellrichtungen E1, E2 und E3 in den Figuren 2A und 2B gezeigt.

Durch diese drei unterschiedlichen Einstellrichtungen E1 bis E3 kann die Position der Sonde 50 in allen drei Freiheitsgraden des Raumes in ihre jeweilige Soll-Sondenposition angeordnet, befestigt und/oder ausgerichtet werden.

Weiterhin kann das Sondenanordenmittel 20 auch um die Befestigungsstange 30 verkippbar ausgebildet sein, also um die zweite Einstellrichtung E2 verschwenkbar. Dadurch ist die Position der Sonde 50 relativ zur Anlage 100 noch einfacher und/oder genauer einstellbar.

Die genaue Sollposition der Sonde 50 und/oder der Sonden 50 kann von einer Bedienperson eingestellt werden. Alternativ oder zusätzlich kann diese Ausbildung des Sondenhalters 1 eine gewisse selbst-Einstellfähigkeit des Sondenhalters 1 bzw. der daran angeordneten Sonde 50 bewirken, der z.B. auf eine Ausdehnung bzw. Verlagerung des Einwegbehälters reagieren kann.

Hierbei kann z.B. ein Einstellen der Sondenposition in die erste Einstellrichtung E1, also in Sondenerstreckungsrichtung S1, automatisch bei einem Ausdehnen und/oder Zusammenziehen der Behälterwand 40 erfolgen, an der die Sonde 50 angedockt und/oder befestigt ist. Ein Einstellen der Sondenposition in die zweite und/oder dritte Einstellrichtung E2, E3 kann z.B. durch eine Bedienperson erfolgen, die das Sondenanordenmittel 20 entsprechend an der Halterungsbasis 10 anordnet und/oder befestigt, insbesondere in der z.B. vertikalen Einstellrichtung E3.

Ein Einstellen der Sondenposition, insbesondere des Neigungswinkels a, durch Verschwenken um die Befestigungsstange 30 kann z.B. ebenfalls automatisch bei einem Ausdehnen und/oder Zusammenziehen der Behälterwand 40 erfolgen, z.B. im Rahmen eines vorbestimmten Spiels, das durch den bzw. die Querschnitt(e) der Befestigungsstange 30 und/oder der Klemmschiene 25 definiert sein kann.

Alternativ kann der Neigungswinkel α der Sonde 50 auch von einer Bedienperson eingestellt werden und/oder fixiert sein.

Im Rahmen dieser Erfindung können die Begriffe "im Wesentlichen" und/oder "etwa" so verwendet sein, dass sie eine Abweichung von bis zu 5% von einem auf den Begriff folgenden Zahlenwert beinhalten, eine Abweichung von bis zu 5° von einer auf den Begriff folgenden Richtung und/oder von einem auf den Begriff folgenden Winkel.

### Bezugszeichenliste

- 1: Sondenhalter

- 10: Halterungsbasis
- 11: erstes, anlagenfestes Bauteil,
- 12: zweites, bewegliches Bauteil
- 13: Anbringmittel
- 13a: Aussparung
- 13b: Schraubmittel
- 14: Stellrad
- 15: Schiene
- 15A: Skala
- 16: Nut
- 17: Block

- 20: Sondenanordenmittel
- 21: Sondenaufnahme
- 22: erster Haltebereich
- 23: zweiter Haltebereich
- 24: Rastmittel
- 25: Klemmschiene
- 26: Stangenaufnahme
- 27: Mittelbereich

- 30: Befestigungsstange

- 40: Behälterwand
- 41: Andockvorrichtung

- 50: Sonde
- 51: Sondenkopf
- 52: Befestigungsende

- 100: Anlage
- 110: Aufnahmebehälter
- 112: Bodenfenster
- 113: Kabelführung
- 114: Rührvorrichtung
- 115: Auffangwanne
- 118: Rollen
- 119: Fixierfüße
- 120: Systemschiene
- 130: Einblatttür
- 132: Türfenster
- 134: Türscharnier
- 136: Türgriff

- E1: erste Einstellrichtung
- E2: zweite Einstellrichtung
- E3: dritte Einstellrichtung
- L: Lot auf die Vertikale
- S: Sondenerstreckungsrichtung
- S1: erste Sondenerstreckungsrichtung
- S2: zweite Sondenerstreckungsrichtung
- S3: dritte Sondenerstreckungsrichtung
- S4: vierte Sondenerstreckungsrichtung
- V: Vertikale

- α: Neigungswinkel

## Patentansprüche

1. Sondenhalter (1) zum Anordnen zumindest einer Sonde (50) derart, dass die Sonde (50) zumindest teilweise in eine Anlage (100) für biotechnologische Anwendungen eingreift, mit:
- einer Halterungsbasis (10) zum Anordnen des Sondenhalters (1) an der Anlage (100) für biotechnologische Anwendungen und
- zumindest einem Sondenanordenmittel (20), das dazu ausgebildet ist, die zumindest eine Sonde (50) so anzuordnen, dass sich die Sonde (50) in eine Sondenerstreckungsrichtung (S; S1, S2, S3, S4) erstreckt;
wobei das Sondenanordenmittel (20) so an der Halterungsbasis (10) angeordnet ist, dass die Position der Sonde (50) in einer Betriebsposition in Sondenerstreckungsrichtung (S; S1, S2, S3, S4) variabel einstellbar ist.

2. Sondenhalter nach Anspruch 1, wobei das Sondenanordenmittel (20) so an der Halterungsbasis (10) angeordnet ist, dass sich die Position der Sonde (50) in Sondenerstreckungsrichtung (S; S1, S2, S3, S4) bei Krafteinwirkung in Sondenerstreckungsrichtung (S; S1, S2, S3, S4) automatisch einstellt.

3. Sondenhalter nach Anspruch 1 oder 2, wobei das Sondenanordenmittel (20) dazu ausgebildet ist, das Gewicht der Sonde (50) im Wesentlichen vollständig zu tragen, wenn die Sonde (50) in ihrer Sondenerstreckungsrichtung (S; S1, S2, S3, S4) angeordnet ist.

4. Sondenhalter nach einem der vorangegangenen Ansprüche, wobei das Sondenanordenmittel (20) so ausgebildet ist, dass es die Sonde (50) so aufnimmt, dass zumindest ein Sondenkopf (51) der Sonde (50) frei aus dem Sondenanordenmittel (20) heraus weist und in Sondenerstreckungsrichtung (S; S1, S2, S3, S4) in Richtung zur Anlage (100) für biotechnologische Anwendungen hin weist;
und/oder
wobei das Sondenanordenmittel (20) dazu ausgebildet ist, die Sonde (50) in einem Formschluss und/oder Klemmsitz aufzunehmen.

5. Sondenhalter nach einem der vorangegangenen Ansprüche, wobei die Halterungsbasis (10) eine Befestigungsstange (30) aufweist, an der das Sondenanordenmittel (20) so angeordnet ist, dass die Position der Sonde (50) in Sondenerstreckungsrichtung (S; S1, S2, S3, S4) variabel einstellbar ist.

6. Sondenhalter nach Anspruch 5, wobei das Sondenanordenmittel (20) in Sondenerstreckungsrichtung (S; S1, S2, S3, S4) verschiebbar, in eine Stangenerstreckungsrichtung der Befestigungsstange (30) verschiebbar und/oder um die Befestigungsstange (30) verschwenkbar an der Befestigungsstange (30) angeordnet ist.

7. Sondenhalter nach Anspruch 5 oder 6, wobei die Befestigungsstange (30) zumindest in einem Befestigungsbereich, an dem das Sondenanordenmittel (20) angeordnet ist, im Wesentlichen orthogonal zur Sondenerstreckungsrichtung (S; S1, S2, S3, S4) angeordnet ist.

8. Sondenhalter nach einem der Ansprüche 5 bis 7, wobei das Sondenanordenmittel (20) ein Befestigungsmittel (25) aufweist, mit dem es mittels Formschluss und/oder Klemmsitz an der Befestigungsstange (30) befestigt ist.

9. Sondenhalter nach einem der Ansprüche 5 bis 8, wobei an der Befestigungsstange (30) eine Mehrzahl von Sondenanordenmittel (20) zum Anordnen jeweils zumindest einer Sonde (50) so angeordnet ist, dass die Position der jeweiligen Sonde (50) in ihrer jeweiligen Sondenerstreckungsrichtung (S; S1, S2, S3, S4) variabel einstellbar ist.

10. Sondenhalter nach einem der Ansprüche 5 bis 9, wobei die Halterungsbasis (10) zumindest zwei Befestigungsstangen (30) aufweist, an denen jeweils zumindest ein Sondenanordenmittel (20) zum Anordnen jeweils zumindest einer Sonde (50) so angeordnet ist, dass eine Position der jeweiligen Sonde (50) in ihrer jeweiligen Sondenerstreckungsrichtung (S; S1, S2, S3, S4) variabel einstellbar ist.

11. Sondenhalter nach einem der vorangegangenen Ansprüche, wobei die Halterungsbasis (10) so an der Anlage für biotechnologische Anwendungen angeordnet ist, dass eine Position des Sondenanordenmittels (20) in einer Außenflächenrichtung der Anlage (100) für biotechnologische Anwendungen variabel einstellbar ist, wobei die Außenflächenrichtung etwa parallel zu einer Außenfläche der Anlage (100) für biotechnologische Anwendungen angeordnet ist und einen Winkel von zumindest etwa 45° zu der Sondenerstreckungsrichtung (S; S1, S2, S3, S4) bildet.

12. Sondenhalter nach Anspruch 11, wobei die Halterungsbasis (10) eine Nut (16) und eine in die Nut (16) eingreifende Feder aufweist, wobei sich die Nut (16) und die Feder in etwa parallel zueinander in etwa in die Außenflächenrichtung erstrecken.

13. Anlage (100) für biotechnologische Anwendungen zum Aufnehmen eines Einwegbehälters mit einem Sondenhalter (1) nach einem der vorangegangenen Ansprüche, wobei im Sondenhalter (1) die zumindest eine Sonde (50) in Sondenerstreckungsrichtung (S; S1, S2, S3, S4) angeordnet ist.

14. Anlage (100) für biotechnologische Anwendungen nach Anspruch 13, wobei in der Anlage (100) für biotechnologische Anwendungen der Einwegbehälter angeordnet ist und ein Sondenkopf (51) der Sonde (50) durch eine Behälterwand (40) des Einwegbehälters hindurch ins Innere des Einwegbehälters eingreift;
und/oder
wobei in der Anlage (100) für biotechnologische Anwendungen der Einwegbehälter angeordnet ist und die Sonde (50) fest an einer Behälterwand (40) des Einwegbehälters befestigt ist.

15. Verfahren zum Anordnen zumindest einer Sonde (50) derart, dass die Sonde (50) zumindest teilweise in eine Anlage (100) für biotechnologische Anwendungen eingreift, mit den Schritten:
- Bereitstellen einer Halterungsbasis (10) zum Anordnen eines Sondenhalters (1) an der Anlage (100) für biotechnologische Anwendungen;
- Anordnen einer Sonde (50) in einem Sondenanordenmittel (20), welches an der Halterungsbasis (10) angeordnet ist, so dass sich die Sonde (50) in eine Sondenerstreckungsrichtung (S; S1, S2, S3, S4) erstreckt; und
- variables Einstellen einer Betriebsposition der Sonde (50) in Sondenerstreckungsrichtung (S; S1, S2, S3, S4).

## Claims

1. A probe holder (1) for positioning at least one probe (50) such that the probe (50) engages at least partially in a system (100) for biotechnological applications having:
- a holder base (10) for positioning the probe holder (1) on the system (100) for biotechnological applications and
- at least one probe positioning means (20), that is configured to position the at least one probe (50) such that the probe (50) extends in a probe extension direction (S; S1, S2, S3, S4);
wherein the probe positioning means (20) is arranged on the holder base (10) such that the position of the probe (50) in an operating position in the probe extension direction (S; S1, S2, S3, S4) is variably adjustable.

2. The probe holder according to claim 1, wherein the probe positioning means (20) is positioned on the holder base (10) such that the position of the probe (50) in the probe extension direction (S; S1, S2, S3, S4) automatically adjusts in the event of applied force in the probe extension direction (S; S1, S2, S3, S4).

3. The probe holder according to claim 1 or 2, wherein the probe positioning means (20) is configured to substantially completely bear the weight of the probe (50) when the probe (50) is positioned in its probe extension direction (S; S1, S2, S3, S4).

4. The probe holder according to any of the preceding claims, wherein the probe positioning means (20) is configured such that it receives the probe (50) such that at least one probe head (51) of the probe (50) points freely from the probe positioning means (20) and in the probe extension direction (S; S1, S2, S3, S4) points in the direction of the system (100) for biotechnological applications;
and/or
wherein the probe positioning means (20) is configured to receive the probe (50) in a form closure and/or clamping seat.

5. The probe holder according to any of the preceding claims, wherein the holder base (10) has a fastening rod (30) on which the probe positioning means (20) is positioned such that the position of the probe (50) in the probe extension direction (S; S1, S2, S3, S4) is variably adjustable.

6. The probe holder according to claim 5, wherein the probe positioning means (20) can be moved in the probe extension direction (S; S1, S2, S3, S4) of the fastening rod (30), can be moved in a rod extension direction of the fastening rod (30) and/or is pivotably arranged on the fastening rod (30).

7. The probe holder according to claim 5 or 6, wherein the fastening rod (30) is positioned substantially orthogonally to the probe extension direction (S; S1, S2, S3, S4) at least in a fastening region at which the probe positioning means (20) is positioned.

8. The probe holder according to any of claims 5 to 7, wherein the probe positioning means (20) has a fastening means (25) with which it is fastened on the fastening rod (30) by means of form closure and/or clamping seat.

9. The probe holder according to any of claims 5 to 8, wherein a plurality of probe positioning means (20) for positioning at least one probe (50) each is positioned on the fastening rod (30) such that the position of the respective probe (50) in its respective probe extension direction (S; S1, S2, S3, S4) is variably adjustable.

10. The probe holder according to any of claims 5 to 9, wherein the holder base (10) has at least two fastening rods (30), at which in each case at least one probe positioning means (20) for positioning in each case at least one probe (50) is positioned such that a position of the respective probe (50) in its respective probe extension direction (S; S1, S2, S3, S4) is variably adjustable.

11. The probe holder according to any of the preceding claims, wherein the holder base (10) is positioned on the system for biotechnological applications such that a position of the probe positioning means (20) in an external surface direction of the system (100) for biotechnological applications is variably adjustable, wherein the external surface direction is positioned roughly parallel to an external surface of the system (100) for biotechnological applications and forms an angle of at least 45° to the probe extension direction (S; S1, S2, S3, S4).

12. The probe holder according to claim 11, wherein the holder base (10) has a groove (16) and a spring engaging in the groove (16), wherein the groove (16) and the spring extend roughly parallel to each other roughly in the external surface direction.

13. A system (100) for biotechnological applications for receiving a disposable container with a probe holder (1) according to any of the preceding claims, wherein the at least one probe (50) is positioned in the probe extension direction (S; S1, S2, S3, S4) in the probe holder (1).

14. The system (100) for biotechnological applications according to claim 13, wherein the disposable container is positioned in the system (100) for biotechnological applications and a probe head (51) of the probe (50) engages through a container wall (40) of the disposable container into the interior of the disposable container; and/or wherein the disposable container is positioned in the system (100) for biotechnological applications and the probe (50) is firmly fastened on a container wall (40) of the disposable container.

15. A method for positioning at least one probe (50) such that the probe (50) at least partially engages in a system (100) for biotechnological applications, having the steps:
- providing a holder base (10) for positioning a probe holder (1) on the system (100) for biotechnological applications;
- positioning a probe (50) in a probe positioning means (20), which is positioned on the holder base (10) so that the probe (50) extends in a probe extension direction (S; S1, S2, S3, S4); and
- variable adjustment of an operating position of the probe (50) in probe extension direction (S; S1, S2, S3, S4).

## Revendications

1. Porte-sonde (1) pour disposer au moins une sonde (50), de telle sorte que la sonde (50) s'insère au moins en partie dans une installation (100) pour applications biotechnologiques, avec :
- une base de support (10) pour la disposition du porte-sonde (1) sur l'installation (100) pour applications biotechnologiques et
- au moins un moyen de disposition de sonde (20), qui est réalisé pour disposer la au moins une sonde (50), de sorte que la sonde (50) s'étend dans une direction d'étendue de sonde (S ; S1, S2, S3, S4) ;
dans lequel le moyen de disposition de sonde (20) est disposé sur la base de support (10), de sorte que la position de la sonde (50), dans une position de fonctionnement, peut être réglée de façon variable dans la direction d'étendue de sonde (S ; S1, S2, S3, S4).

2. Porte-sonde selon la revendication 1, dans lequel le moyen de disposition de sonde (20) est disposé sur la base de support (10) de sorte que la position de la sonde (50) se règle automatiquement dans la direction d'étendue de sonde (S ; S1, S2, S3, S4) lorsqu'une force est exercée dans la direction d'étendue de sonde (S ; S1, S2, S3, S4).

3. Porte-sonde selon la revendication 1 ou 2, dans lequel le moyen de disposition de sonde (20) est réalisé pour porter sensiblement entièrement le poids de la sonde (50), lorsque la sonde (50) est disposée dans sa direction d'étendue de sonde (S ; S1, S2, S3, S4).

4. Porte-sonde selon l'une quelconque des revendications précédentes, dans lequel le moyen de disposition de sonde (20) est réalisé de sorte qu'il reçoit la sonde (50), de sorte qu'au moins une tête de sonde (51) de la sonde (50) est dirigée librement à l'extérieur du moyen de disposition de sonde (20) et, dans la direction d'étendue de sonde (S ; S1, S2, S3, S4), est dirigée en direction de l'installation (100) pour applications biotechnologiques ;
et/ou
dans lequel le moyen de disposition de sonde (20) est réalisé pour recevoir la sonde (50) dans une complémentarité de formes et/ou un ajustement serré.

5. Porte-sonde selon l'une quelconque des revendications précédentes, dans lequel la base de support (10) présente une tige de fixation (30) sur laquelle le moyen de disposition de sonde (20) est disposé, de sorte que la position de la sonde (50) peut être réglée de façon variable dans la direction d'étendue de sonde (S ; S1, S2, S3, S4).

6. Porte-sonde selon la revendication 5, dans lequel le moyen de disposition de sonde (20) est disposé sur la tige de fixation (30) de manière à pouvoir se déplacer dans la direction d'étendue de sonde (S ; S1, S2, S3, S4), à pouvoir se déplacer dans une direction d'étendue de tige de la tige de fixation (30) et/ou à pouvoir pivoter autour de la tige de fixation (30).

7. Porte-sonde selon la revendication 5 ou 6, dans lequel la tige de fixation (30) est disposée au moins dans une zone de fixation, sur laquelle le moyen de disposition de sonde (20) est disposé, sensiblement perpendiculairement à la direction d'étendue de sonde (S ; S1, S2, S3, S4).

8. Porte-sonde selon l'une quelconque des revendications 5 à 7, dans lequel le moyen de disposition de sonde (20) présente un moyen de fixation (25), avec lequel il est fixé à la tige de fixation (30) au moyen d'une coopération de formes et/ou d'un ajustement serré.

9. Porte-sonde selon l'une quelconque des revendications 5 à 8, dans lequel une pluralité de moyens de disposition de sonde (20) pour disposer respectivement au moins une sonde (50) est disposée sur la tige de fixation (30), de sorte que la position de la sonde (50) respective peut être réglée de façon variable dans la direction d'étendue de sonde (S ; S1, S2, S3, S4) respective de celle-ci.

10. Porte-sonde selon l'une quelconque des revendications 5 à 9, dans lequel la base de support (10) présente au moins deux tiges de fixation (30), sur lesquelles respectivement au moins un moyen de disposition de sonde (20) pour disposer respectivement au moins une sonde (50) est disposé, de sorte qu'une position de la sonde (50) respective peut être réglée de façon variable dans la direction d'étendue de sonde (S ; S1, S2, S3, S4) respective de celle-ci.

11. Porte-sonde selon l'une quelconque des revendications précédentes, dans lequel la base de support (10) est disposée sur l'installation pour applications biotechnologiques de sorte qu'une position du moyen de disposition de sonde (20) peut être réglée de façon variable dans une direction de surface extérieure de l'installation (100) pour applications biotechnologiques, dans lequel la direction de surface extérieure est disposée approximativement parallèlement à une surface extérieure de l'installation (100) pour applications biotechnologiques et forme un angle d'au moins approximativement 45° par rapport à la direction d'étendue de sonde (S ; S1, S2, S3, S4).

12. Porte-sonde selon la revendication 11, dans lequel la base de support (10) présente une rainure (16) et un ressort s'insérant dans la rainure (16), dans lequel la rainure (16) et le ressort s'étendent à peu près parallèlement l'une à l'autre à peu près dans la direction de surface extérieure.

13. Installation (100) pour applications biotechnologiques pour recevoir un contenant jetable avec un porte-sonde (1) selon l'une quelconque des revendications précédentes, dans laquelle la au moins une sonde (50) est disposée dans le porte-sonde (1) dans la direction d'étendue de sonde (S ; S1, S2, S3, S4).

14. Installation (100) pour applications biotechnologiques selon la revendication 13, dans laquelle le contenant jetable est disposé dans l'installation (100) pour applications biotechnologiques et une tête de sonde (51) de la sonde (50) s'insère à l'intérieur du contenant jetable à travers une paroi de contenant (40) du contenant jetable ;
et/ou
dans laquelle le contenant jetable est disposé dans l'installation (100) pour applications biotechnologiques et la sonde (50) est fixée solidement à une paroi de contenant (40) du contenant jetable.

15. Procédé pour disposer au moins une sonde (50), de telle sorte que la sonde (50) s'insère au moins en partie dans une installation (100) pour applications biotechnologiques, avec les étapes :
- de fourniture d'une base de support (10) pour disposer un porte-sonde (1) sur l'installation (100) pour applications biotechnologiques ;
- de disposition d'une sonde (50) dans un moyen de disposition de sonde (20), lequel est disposé sur la base de support (10), de sorte que la sonde (50) s'étend dans une direction d'étendue de sonde (S ; S1, S2, S3, S4) ; et
- de réglage variable d'une position de fonctionnement de la sonde (50) dans la direction d'étendue de sonde (S ; S1, S2, S3, S4).
